# EUROPÄISCHE PATENTSCHRIFT

(11) **EP 1 764 133 B1**
(45) Veröffentlichungstag und Bekanntmachung des Hinweises auf die Patenterteilung: **17.08.2011**
(21) Anmeldenummer: 06119988.1
(22) Anmeldetag: 01.09.2006
(51) Int. Cl.: A61N 5/10

(54) **Partikeltherapieanlage und Verfahren zur Ausbildung eines Strahlpfads für einen Bestrahlungsvorgang in einer Partikeltherapieanlage**
Particle therapy system and method for arranging a radiation beam for performing a particle therapy process in a particle therapy system
Système et procédé de thérapie par particules pour la formation d'une trajectoire d'un rayon pour un processus d'irradiation dans un système de thérapie par particules

(30) Priorität: 16.09.2005 DE 102005044409; 16.09.2005 US 717835 P
(43) Veröffentlichungstag der Anmeldung: 21.03.2007
(73) Patentinhaber: SIEMENS AKTIENGESELLSCHAFT, 80333 München (DE)
(72) Erfinder: Birgy, Denis, 91091, Großenseebach (DE); Breuninger, Harald, 91330, Eggolsheim (DE)

(56) Entgegenhaltungen:
- EP-A- 1 454 657
- US-A- 5 260 581
- US-A1- 2005 029 472

## Beschreibung

Die Erfindung betrifft eine Partikeltherapieanlage mit einer Beschleuniger- und Partikelstrahlzuführungseinheit zur Beschleunigung von Partikeln und zur Zuführung von Partikeln an mindestens zwei Bestrahlungsplätzen und mit einer einem Kontroll- und Sicherheitssystem zur Sicherstellung und Überwachung der korrekten Partikelstrahlführung.

Eine Partikeltherapieanlage weist üblicherweise eine Partikelbeschleunigereinheit, eine sich anschließenden Partikelstrahlzuführungseinheit sowie mehrere Bestrahlungsplätze auf. Die Beschleunigung der Partikel, z.B. Protonen, Pionen, Helium-, Kohlenstoff- oder Sauerstoff-Ionen, erfolgt beispielsweise mit Hilfe eines Synchrotrons oder Zyklotrons. Die beschleunigten hochenergetischen Partikel werden aus der Partikelbeschleunigereinheit ausgekoppelt und in die auch als Hochenergiestrahltransportsystem (high energy beam transport system HEBT) bezeichnete Partikelstrahlzuführungseinheit eingekoppelt. Im Falle eines Synchrotrons erfolgt die Auskopplung beispielsweise über einen Knock-Out-Exciter. Die HEBT erlaubt es, die hochenergetischen Partikel immer demjenigen Bestrahlungsplatz zuzuführen, an dem gerade ein Bestrahlungsvorgang erfolgen soll.

An einem im Folgenden auch als Behandlungsplatz bezeichneten Bestrahlungsplatz erfolgt z.B. eine Tumortherapie eines Patienten, der dazu im Partikelstrahlengang positioniert und den hochenergetischen Partikel ausgesetzt wird. Man unterscheidet zwischen "fixed beam" Behandlungsplätzen, in denen die Partikel aus einer festen Richtung auf einen Behandlungsplatz treffen, und so genannten Gantry basierten Behandlungsplätzen. Bei letzteren ist es möglich, den Partikelstrahl aus verschiedenen Richtungen auf den Behandlungsplatz der Gantry zu richten. Ferner erfolgt eine Überwachung der Strahlqualität an einem im Folgenden als Überprüfungsplatz bezeichneten Bestrahlungsplatz. Dort werden Strahlparameter wie Partikelenergie, Energieverteilung und Strahlintensität in Qualitätsmessungen überwacht.

An die Sicherheit einer Partikeltherapieanlage sind hohe Ansprüche gestellt. So muss z.B. gewährleistet sein, dass der Partikelstrahl immer nur zu einem Bestrahlungsplatz geführt wird, der auf einen Bestrahlungsvorgang vorbereitet ist und der den Partikelstrahl angefordert hat. Ferner muss gewährleistet sein, dass der Partikelstrahl die korrekten angeforderten Parameter aufweist. Ferner ist im Notfall eine schnelle Unterbrechung der Partikelzuführung notwendig. Dazu weist z.B. die HEBT eine Schikane auf, die es erlaubt den Partikelstrahl schnell abzuschalten. Üblicherweise gewährleistet ein Kontroll- und Sicherheitssystem der Partikeltherapieanlage, dass jeweils ein mit den benötigten Parametern charakterisierter Partikelstrahl in den entsprechenden Behandlungsraum geführt wird.

Die benötigten Parameter werden im so genannten Behandlungs- oder Therapieplan definiert. Dieser gibt an, wie viele Teilchen aus welcher Richtung mit welcher Energie auf den Patienten treffen sollen. Die Energie der Partikel bestimmt die Eindringtiefe der Partikel in den Patienten, d.h. den Ort, an dem das Maximum der Wechselwirkung mit dem Gewebe bei der Partikeltherapie erfolgt; in anderen Worten, den Ort, an dem das Maximum der Dosis deponiert wird. Die vom Therapieplan geforderten Parameter werden üblicherweise von einer Beschleunigerkontrolleinheit in Einstellparameter, z.B. in Form von Maschinenparametern, für die Beschleuniger- und Partikelstrahlzuführungseinheit umgesetzt. Ferner wird die Information, an welchen Bestrahlungsplatz der Partikelstrahl geführt werden soll, in Einstellparameter für die Partikelstrahlzuführungseinheit umgesetzt. Des Weiteren steuert eine Kontrolleinheit des Bestrahlungsplatzes beispielsweise eine Positioniervorrichtung, mit der ein zu bestrahlender Patient/ein zu bestrahlendes Phantom in Bezug zum Partikelstrahl positioniert wird.

Eine Partikeltherapieanlage mit mehreren Fixed-Beam-Behandlungsplätzen und einer Gantry ist z.B. aus EP 0 986 070 bekannt. Verschiedene Bestrahlungsanlagen und -techniken sind von H. Blattmann in "Beam delivery systems for charged particles", Radiat. Environ. Biophys. (1992) 31:219-231 beschrieben.

Ein Verfahren zur Auswahl eines Behandlungsraumes ist z.B. aus US 5,260,581 und ein Kontroll- und Sicherheitssystem für eine Strahlentherapieanlage ist z.B. aus US 5,895,926 bekannt.

Der Erfindung liegt die Aufgabe zugrunde, einen sicheren Betrieb einer Partikeltherapieanlage und insbesondere eine sichere Zuführung eines Partikelstrahls an einen den Partikelstrahl anfordernden Bestrahlungsplatz zu gewährleisten.

Diese Aufgabe wird erfindungsgemäß gelöst durch eine Partikeltherapieanlage nach Anspruch 1 sowie durch ein Verfahren nach Anspruch 14.

Die spezielle Untergliederung des Kontroll- und Sicherheitssystem zur Sicherstellung und Überwachung der korrekten Partikelstrahlführung entlang eines Strahlpfades, in eine Zuordnungseinheit, mindestens eine an einem der Bestrahlungsplätze angeordnete Kontrolleinheit und eine Beschleunigerkontrolleinheit erlaubt die vorteilhafte Trennung der Steuerung und Kontrolle von therapienahen Vorgängen am Bestrahlungsplatz und die Steuerung und Kontrolle aller Vorgänge und Einstellungen in der Beschleuniger- und Strahlzuführungseinheit.

Wichtig für den sicheren Betrieb einer Partikeltherapieanlage ist die sichere Zuführung des Partikelstrahls an nur darauf vorbereitete Bestrahlungsräume. Besonders vorteilhaft kann die Sicherheit durch die zwischengeschaltete Zuordnungseinheit gewährleistet werden. Sie empfängt die Anforderungssignale von den Kontrolleinheiten der Bestrahlungsräume, koordiniert die Reihenfolge von Bestrahlungsvorgängen und ordnet bei entsprechender Verfügbarkeit des Partikelstrahls diesen dem anfordernden Bestrahlungsraum zu. Dies erfolgt über den direkten Austausch von Anforderungs- und Bestätigungssignalen in der Anforderungsphase. In der Einstellungsphase findet die Übermittlung von für den Bestrahlungsvorgang wichtiger Information statt - z.B. von der Kontrolleinheit über ein BUSSystem zur Beschleunigerkontrolleinheit. Die Beschleunigerkontrolleinheit übermittelt dann für den Bestrahlungsvorgang benötigte Einstellparameter an die den Strahlpfad festlegenden Elemente der Beschleuniger- und Strahlzuführungseinheit. Eine Umsetzung der Einstellparameter kann nur bei Vorliegen eines Aktivierungssignals von der Zuordnungseinheit erfolgen. Diese übermittelt in der Aktivierungsphase die Zuordnungseinheit direkt an die für den entsprechenden Strahlpfad benötigten einstellbaren Elemente. Dazu weist die beispielsweise als sicherheitsgerichtete Steuereinheit ausgebildete Zuordnungseinheit einen Speicher mit einem Look-Up-Table auf, der Information darüber aufweist, welche Elemente für welchen Bestrahlungsplatz zu aktivieren sind. Dieser von der Beschleunigerkontrolleinheit entkoppelte Aktivierungsschritt bietet doppelte Sicherheit bei der Einstellung des Strahlpfads. Vorzugsweise werden direkte und fest zugeordnete Signalverbindungen verwendet. Eine direkte und fest zugeordnete Signalverbindung ist z.B. eine direkte Hardware-Verbindung, insbesondere eine einzelne und bevorzugt eine sicherheitsgerichtete, Signalleitung. Dabei schließt direkt auch ein Zusammenklemmen von mehreren Kabelabschnitten ein, wobei ein einziges durchgehend verlegtes Kabel bevorzugt ist.

Eine direkte und fest zugeordnete Signalverbindung hat den Vorteil, dass eine eindeutige Zuordnung eines Bestrahlungsplatzes zu einem Signaleingang besteht. Dadurch ist es gewährleistet, dass immer der richtige Bestrahlungsplatz von der Zuordnungseinheit erkannt wird, ohne dass hier eine Bestätigung zur Verifikation des richtigen Bestrahlungsplatzes über z.B. ein Protokoll benötigt wird. Es besteht also eine Sicherheit bei der Zuordnung des Bestrahlungsplatzes ohne zusätzlichen Verifikationsschritt. Somit erlaubt eine auf diese Weise Hardware kodierte und kontrollierte Vorgehensweise ein eine gesicherte Zuführung eines Partikelstrahls entlang eines Partikelpfads zum anfordernden Bestrahlungsplatz. Die Signalverbindung wird vorzugsweise nur in eine Richtung verwendet, so dass eine störanfällige Logik zur Differenzierung der Richtung der Signalübertragung nicht benötigt wird.

Das Beruhen des Sicherheitskonzepts auf einer Hardware-Kodierung ist ein Vorteil gegenüber dem Verfahren in der eingangs genannten Schrift US 5,895,926. Denn eine gesicherte Zuordnung und/oder eine gesicherte Strahlverfügbarkeitskontrolle durch dedizierte Hardware-Signalleitungen erschwert Manipulationsmöglichkeiten gegenüber einer reinen Bus-Lösung, denen eine variable Signalgebung aufprägbar ist.

Eine Ausführungsform einer erfindungsgemäßen Partikeltherapieanlage weist beispielsweise eine Beschleuniger- und Partikelstrahlzuführungseinheit zur Beschleunigung von Partikeln und zur Zuführung von Partikeln vom Beschleuniger an mindestens zwei Bestrahlungsplätze auf. Eine derartige Einheit umfasst beispielsweise als Beschleuniger ein Zyklotron oder eine Synchrotron, in das evtl. vorbeschleunigte Partikel eingekoppelt werden. Die Partikelstrahlzuführung erfolgt z.B. mit Hilfe von mindestens einem einstellbaren Element im Strahlpfad. Das oder die Elemente werden mit Hilfe der Beschleunigerkontrolleinheit entsprechend dem jeweils benötigten Strahlpfad eingestellt. Zur Einstellung benötigte Einstellparameter werden übermittelt und z.B. in einem Zwischenspeicher abgelegt.

Mindestens einer der Behandlungsplätze weist eine Kontrolleinheit auf, die über einer direkte und fest zugeordnete Signalverbindung direkt mit einem Signaleingang der Zuordnungseinheit verbunden ist und die die zur Abgabe eines Anforderungssignals zur Anforderung eines Partikelstrahls für einen Bestrahlungsvorgang über die Signalverbindung ausgebildet ist, so dass ein Anliegen des Anforderungssignals am Signaleingang eindeutig den anfordernden Bestrahlungsplatz festlegt.

Vorzugsweise besteht zwischen der Zuordnungseinheit und der Kontrolleinheit des Bestrahlungsplatzes eine zweite fest zugeordnete Signalverbindung zur Übermittlung eines Bestätigungssignals von einem Signalausgang der Zuordnungseinheit zur Kontrolleinheit.

Üblicherweise wird die Therapieanlage mehrere Bestrahlungsplätze aufweisen. Diese sind jeweils einzeln über fest zugeordnete Signalverbindung direkt mit je einem Signaleingang (und evtl. Signalausgang) der Zuordnungseinheit verbunden. Damit sind die Kontrolleinheiten über eine direkte Hardwareverbindung beispielsweise durch einzelne, direkte Signalleitungen mit der Zuordnungseinheit verbunden.

Üblicherweise wird die Beschleuniger- und Partikelstrahlzuführungseinheit mehrere Elemente aufweisen. Diese sind z.B. ebenfalls jeweils einzeln über je eine fest zugeordnete Signalverbindung direkt mit je einem Signalausgang der Zuordnungseinheit verbunden. Damit sind z.B. auch die einstellbaren Elemente über eine direkte Hardwareverbindung beispielsweise durch einzelne, direkte Signalleitungen mit der Zuordnungseinheit verbunden.

Beispiele für einstellbare Elemente sind Strahlumlenkmagnete, die den Partikelstrahl von dem Strahlzuführungssystem in die einzelnen Behandlungsräume ablenken, eine Strahlauskopplungsvorrichtung eines Beschleunigers, beispielsweise ein Knock-Out-Exciter eines Synchrotron-Rings, ein Dipolmagnet einer Schikane im HEBT. Mögliche Einstellparameter sind entsprechend das anliegende Magnetfeld, ein dafür benötigter einzustellender Stromwert oder eine HF-Auskoppelfrequenz. Ein einstellbares Element ist vorzugsweise zur Verarbeitung und in Abhängigkeit vom Vorliegen des Aktivierungssignals zur Umsetzung des mindestens einen übermittelten Einstellparameters ausgebildet. Dazu weist es beispielsweise einen Pufferspeicher auf, in den ein übermittelter Einstellparameter abgelegt und nach Erhalt des Aktivierungssignals ausgelesen werden kann.

Eine Übermittlung der Einstellparameter erfolgt beispielsweise über ein Datenbus-System, an das die Beschleunigerkontrolleinheit und die jeweiligen Element angebunden sind. Der Einstellparameter wird durch den am Bestrahlungsplatz stattfindenden Bestrahlungsvorgang bedingt.

Ebenfalls vorteilhaft ist es, wenn mindestens einer Kontrolleinheit der Bestrahlungsplätze zum Austausch von zur Bestrahlung benötigten Parametern des Partikelstrahls und/oder Parameter der Beschleuniger- und Partikelstrahlzuführungseinheit an dieses Datenbus-System oder an einem eigenen Datenbus-System angebunden sind.

Beispiele für Bestrahlungsplätze sind ein Behandlungsplatz zur Strahlentherapie, z.B. ein Fixed-Beam- oder Gantry-Behandlungsplatz, oder ein Überprüfungsplatz zur Überprüfung von der Partikelbestrahlung zugrunde liegenden Parametern.

Ferner kann bei Eintreten eines Fehlers die Partikelbeschleunigung und/oder einer Weiterleitung der Partikel beispielsweise von der Zuordnungseinheit und/oder der Kontrolleinheit unterbrochen werden. Dazu wird, falls beispielsweise für ein Einstellen des Elements fortwährend ein Aktivierungssignal vorliegen muss, dieses Aktivierungssignal beendet. Wirkt das Aktivierungssignal als Schalter, kann des Element auf "nichteinstellbar" geschaltet werden.

Weitere vorteilhafte Ausführungsformen der Erfindung sind durch die Merkmale der Unteransprüche gekennzeichnet.

Es folgt die Erläuterung von mehreren Gesichtspunkten der Erfindung anhand der Figuren 1 bis 3. Es zeigen:
Figur 1 einen schematischen Überblick über eine Therapieanlage,
Figur 2 eine schematische sicherheitsgerichtete Schalteinheit und
Figur 3 eine Skizze zur Anordnung von sicherheitsgerichteten Verbindungen.

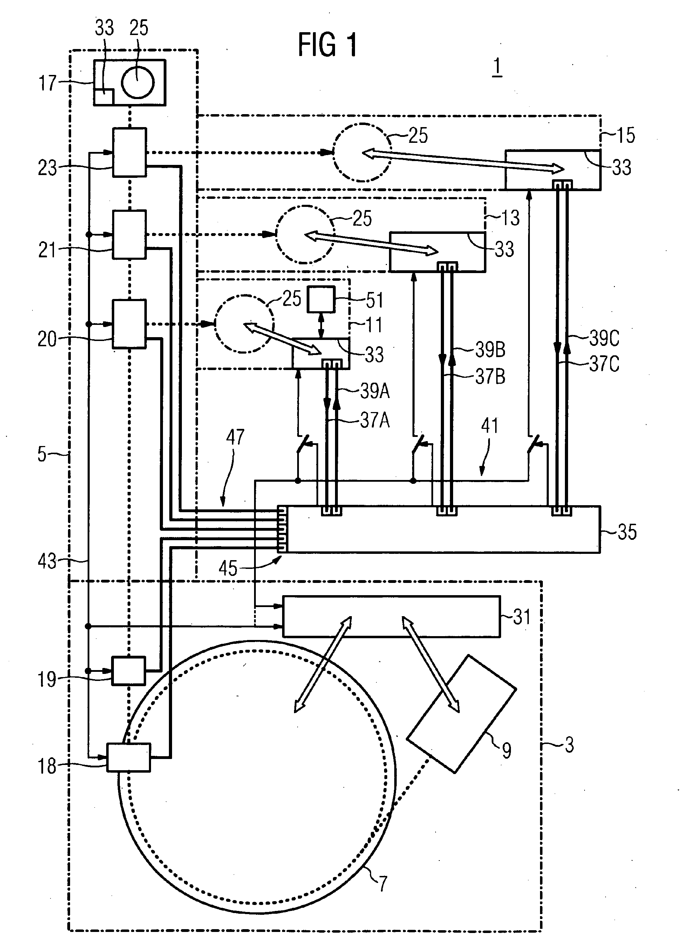

Figur 1 zeigt schematisch eine Therapieanlage 1 und verdeutlicht das Zusammenspiel von verschiedenen involvierten Kontrolleinheiten. Diese bewirkten und überwachen die Einstellung von Komponenten, um einen Strahl mit entsprechenden Parametern an einen Bestrahlungsplatz zu senden. Aus Sicherheitsgründen werden dabei wichtige Signale über Fehler unanfällige Hardware-Verbindung übermittelt. Die Hardware-Verbindung besteht z.B. aus einer eigenen, separaten spezifischen Leitung und ist eindeutig der Übertragung eines Signals zugeordnet.

Die Therapieanlage 1 weist eine Beschleunigereinheit 3 und eine Partikelstrahlzuführungseinheit 5 auf. Beispielhaft für einen Beschleuniger wurde ein Synchrotron 7 mit einer vorgeschaltenen Linearbeschleunigereinheit 9 dargestellt. Die Strahlzuführungseinheit 5 verteilt die Partikel auf mehrere Bestrahlungsplätze. Beispielhaft sind schematisch drei Behandlungsplätze 11, 13 und 15 zur Strahlentherapie und ein Überprüfungsplatz 17 zur Gewährleistung der Qualität des Partikelstrahls angedeutet. Am Überprüfungsplatz 17 erfolgt beispielsweise die Qualitätssicherung mit Hilfe von Qualitätsprozeduren, beispielsweise mit regelmäßigen Tests zur Verifikation der zuvor definierten Strahlparameter, beispielsweise von Positions- und Intensitätsabstufungen, von Partikelenergien. Letztere sind beispielsweise in einer Bibliothek enthalten und werden durch automatisierte Bragg-Peak-Messungen an Phantomen überprüft.

Mithilfe einer Auskoppelvorrichtung 18 werden im beispielsweise Synchrotronring 7 gespeicherte Partikel ausgekoppelt und in die Strahlzuführungseinheit 5 eingekoppelt. Die Möglichkeit einer schnellen Strahlabschaltung nach Beendigung oder Unterbrechung des Bestrahlungsvorgangs erfolgt beispielsweise durch den Einbau einer Schikane 19, welche beispielsweise aus drei kleinen Dipol-Magneten besteht, die nach der Extraktionseinheit 18 angeordnet sind. Durch eine schnelle Abschaltung des beispielsweise mittleren Dipols wird der Strahl an einem Kollimator vernichtet.

Die Zuführung der Partikel zu den Bestrahlungsplätzen 11, 13 und 15 erfolgt durch die Ablenkung des Partikelstrahls mittels Umlenkmagnete 20, 21 und 23 aus einer Hauptstrahlrichtung in der Strahlzuführungseinheit 5. In Hauptstrahlrichtung befindet sich der Überprüfungsplatz 17. An den Bestrahlungsplätzen erfolgt die Wechselwirkung der Partikel mit einem zu bestrahlenden Patienten oder einem Phantom in Bestrahlungszonen 25. Eine der Bestrahlungszonen 25 ist z.B. durch einen maximal abscanbaren Scanbereich einer (Raster-)Scanvorrichtung, einen maximal bestrahlbaren Scatterbereich einer Scattervorrichtung oder einen einstellbaren Gantry-Bestrahlungsbereich etc. gegeben.

Die Linearbeschleunigereinheit 9 kann beispielsweise ein oder mehrere betreibbare Ionenquellen, eine Niedrigenergiestrahlführung, einen Radio-Frequenz-Quadrupol, einen Drift-Röhrenbeschleuniger und eine Injektionsstrahlführung aufweisen. Aufgaben der Injektoreinheit 9 sind die Erzeugung einer oder mehrerer Teilchensorten, deren Befreiung von Verunreinigungen durch nicht gewünschte Teilchensorten, die Einstellung der Strahlintensität im Niedrigenergiebereich beispielsweise für das Synchrotron, eine Vorbeschleunigung der Partikel und eine Vorbereitung des Partikelstrahls beispielsweise in der Pulslänge und den Strahlparametern entsprechend den Anforderungen des Synchrotrons.

Wird die Therapieanlage 1 zur Bestrahlung mittels Scanning-Verfahren eingesetzt, ist eine langsame Extraktion von Vorteil, die eine optimale Nutzung der beschleunigten Teilchen und eine präzise Strahlüberwachung während der Tumorabtastung ermöglicht. In diesem Fall ist bei Verwendung eines Synchrotrons z.B. eine Strahlextraktion mit einer sog. HF-Knock-Out-Methode von Vorteil, bei der ein Knock-Out-Exciter die Auskoppeleinheit 18 bildet.

Das Kontroll- und Sicherheitssystem der Therapieanlage 1 ist zur Verdeutlichung in mehrere Komponenten aufgeteilt. Eine Aufteilung kann auch anders oder gar nicht vorgenommen werden, solange die verschiedenen Aspekte bei der Überwachung berücksichtigt werden.

In der Ausführung nach Figur 1 sorgt eine Beschleunigerkontrolleinheit 31 dafür, dass der angeforderte Partikelstrahl entsprechend seiner Spezifikation im Behandlungsraum ankommt. An den Bestrahlungsplätzen angeordnete Kontrolleinheiten 33 steuern den Ablauf eines Bestrahlungsvorgangs und sorgen dafür, dass der Partikelstrahl entsprechend einer Bestrahlungsplanung auf einen Patienten trifft.

Ferner umfasst das Kontroll- und Sicherheitssystem eine Zuordnungseinheit 35. Diese gewährleistet es, dass nur derjenige Bestrahlungsplatz 11,...17 einen Partikelstrahl zugeführt bekommt, der ihn auch angefordert hat. Dazu ist die Zuordnungseinheit 35 einerseits mit den Kontrolleinheiten 33 zumindest zur Übermittlung eines Anforderungssignals über eine feste und eindeutig zugeordnete Signalleitung 37A, 37B, 37C verbunden. Ferner kann eine weitere fest zugeordnete Signalleitung 39A, 39B, 39C zwischen der Zuordnungseinheit 35 und den Kontrolleinheiten 33 bestehen. Diese kann beispielsweise zur Übermittlung eines Bestätigungssignals von der Zuordnungseinheit 35 zu demjenigen Bestrahlungsplatz, dem als nächstes der Partikelstrahl zugeführt werden soll, verwendet werden.

Vorzugsweise weist das Kontroll- und Sicherheitssystem mindestens ein Datenbus-System 41 auf, an das die Kontrolleinheiten 33 und die Beschleunigerkontrolleinheit 31 angebunden sind. Es dient beispielsweise der Übermittlung von Einstellparametern für die Beschleunigereinheit 3 und die Partikelstrahlzuführungseinheit 5 für eine nächste durchzuführende Bestrahlung. Vorzugsweise kann die Zuordnungseinheit 35 derart auf das Datenbus-System 41 einwirken, dass nur derjenige Bestrahlungsplatz 11,...17, der ein Bestätigungssignal bekommen hat, Parameter übermitteln kann.

An ein weiteres, eventuell auch mit dem Datenbusses 41 zusammengelegtes, Datenbussystem 43 (gestrichelte Verbindung), sind die Beschleunigerkontrolleinheit 31 und von diesem einstellbare Elementen der Beschleuniger- und Strahlzuführungseinheit angebunden. In der Ausführungsform gemäß Figur 1 sind dies beispielsweise die Auskoppeleinheit 18, die Schikane 19 und die Umlenkmagneten 20, 21, 23. Über das Datenbussystem 43 werden diejenigen Einstellparameter an die Elemente übermittelt, die diese Elemente zur Einstellung des aktuell angeforderten Partikelstrahlpfades und für den Transport der Partikel mit der richtigen Energie etc. benötigen. Ein Strahlpfad bedingt Einstellungen von Elemente im Hochenergiestrahlpfad in Abhängigkeit eines festgelegten Bestrahlungsplatzes.

Allerdings kann eine Umsetzung der Einstellparameter nur dann erfolgen, wenn zusätzlich ein Aktivierungssignal der Zuordnungseinheit 35 an dem jeweils einzustellenden Element vorliegt. Dazu sind die einstellbaren Elemente mit Signalausgängen 45 der Signalzuordnungseinheit 35 über direkte, fest zugeordnete Signalleitungen 47 verbunden.

Die Tatsache, dass Anforderungs- und/oder Aktivierungssignale über spezifische unzweideutige Hardware-Verbindungen gesendet und empfangen werden, reicht aus zu gewährleisten, dass das Anforderungssignal von einem gewissen und bekannten Bestrahlungsplatz gesendet wurde und/oder dass nur explizit aktivierte Elemente zur Festlegung des Strahlpfades eingestellt werden. Es ist nicht möglich, dass Signale fehlerhaft von anderen Bestrahlungsplätzen empfangen oder zu anderen Elementen übermittelt werden.

Im Folgenden wird ein sicherer Ablauf einer Bestrahlung eines Patienten beschrieben, wie er beispielsweise unter Verwendung einer Anlage nach Figur 1 erfolgen kann. In einem Therapieplan 51 wird die Bestrahlung mit all seinen erforderlichen Parametern wie Strahleinfallsrichtung, Strahlintensität, Partikelart, Partikelenergie etc. festgelegt.

Nachdem der Bestrahlungsplan für den Patienten am Bestrahlungsplatz geladen, alle sicherheitstechnischen Voraussetzungen erfüllt und der Patient entsprechend positioniert wurde, fordert ein Therapiekontrollsystem beispielsweise eine Kontrolleinheit der Bestrahlungsplätze 11,... 15 einen Strahl mit den geplanten Parametern für den aktuellen Bestrahlungsplatz an. Vorzugsweise können nur getestete und freigegebene Datensätze von Parametern verwendet und angefordert werden, welche im Beschleunigerkontrollsystem 31 abgespeichert vorliegen.

Für die Strahlanforderung veranlasst ein Bediener die Sendung eines Anforderungssignals von beispielsweise der Kontrolleinheit des Bestrahlungsplatzes 11 entlang der direkten fest zugeordneten Signalleitung 37A an die Zuordnungseinheit 35. Die Zuordnungseinheit 35 überprüft die Verfügbarkeit des Partikelstrahls. Findet noch an einem Nachbarbestrahlungsplatz ein Bestrahlungsvorgang statt, teilt die Zuordnungseinheit erst mit Beendigung dieses Bestrahlungsvorgangs dem anfordernden Behandlungsraum den Partikelstrahl zu. Die Zuordnungseinheit gibt beispielsweise erst zu diesem Zeitpunkt die Verbindung von der Kontrolleinheit 33 des Behandlungsraums 11 zur Beschleunigerkontrolleinheit 31 im Datenbus-System 41 für die Übermittlung der gewünschten Parameter für den folgenden Bestrahlungsvorgang frei.

Zusätzlich sendet die Zuordnungseinheit 35 an die für die Strahlzuführung zum anfordernden Bestrahlungsplatz benötigten einstellbaren Elemente, in diesem Fall die Auskoppeleinheit 18, die Schikane 19 und der Umlenkmagnet 20, über die fest zugeordneten Signalleitungen 47 Aktivierungssignale. Ferner übermittelt die Beschleunigerkontrolleinheit 31 Einstellparameter in diese Elementen. Nur bei Vorlage des Aktivierungssignals können die von der Beschleunigerkontrolleinheit 31 übermittelten Einstellparameter in den Elementen umgesetzt werden und den benötigten Partikelstrahlpfad festlegen. Vorzugsweise sind die einstellbaren Elemente vorrangig deaktiviert. Nur bei Vorliegen eines Aktivierungssignals werden entsprechende Ströme etc. eingestellt. Deaktivierung bedeutet, dass beispielsweise der Defaultwert "Strom auf Null" eingestellt wird. D.h., zur Übermittlung eines Aktivierungssignals ist ein Signalausgang der Zuordnungseinheit über eine direkte und fest zugeordnete Signalverbindung mit mindestens einem der einstellbaren Elemente verbunden. Nur in Zusammenspiel mit dem Aktivierungssignal kann eine Umsetzung übermittelten Einstellparameters im Element erfolgen. D.h., das Aktivierungssignal muss z.B. vor und/oder während der Umsetzung vorliegen. Prinzipiell ist es vorzugsweise so vorgesehen, dass die einstellbaren Elemente vorrangig deaktiviert sind, d.h., die Beschleunigerkontrolleinheit wirkt als Verriegelungsmechanismus. Nur bei Vorliegen eines Aktivierungssignals werden entsprechende Ströme etc. eingestellt. Deaktivierung bedeutet, dass beispielsweise die Defaultwerte eingestellt sind, z.B. kein Stromfluss in den Magnetspulen.

Nach erfolgter Einstellung übermittelt die Zuordnungseinheit 35 entlang der direkten Verbindungsleitung 39A ein Bestätigungssignal. Nach einer evtl. Bestätigung dieses Signals durch den Behandlungsplatz 11 erfolgt die Zuführung von Partikeln zur Bestrahlung im Bestrahlungsbereich 25.

Die Reihenfolge von Einstellvorgängen und Signalübermittlungen ist -bis auf das Vorhandensein eines Aktivierungssignals für die tatsächliche Umsetzung von physikalischen Einstellungen- frei gestaltbar. So kann bei einem alternativen Ablauf beispielsweise direkt nach der Zuordnung des Partikelstrahls an den Behandlungsraum 11 das Bestätigungssignal an den Behandlungsraum 11 entlang der Verbindungsleitung 39A übermittelt werden. Ein aktiv von Seiten der Kontrolleinheit 33 des Behandlungsraums auf das Bestätigungssignal ausgelöstes "Strahl an"-Signal veranlasst Aktivierungssignale von der Zuordnungseinheit sowie die Übermittlung von Einstellparameter von der Beschleunigerkontrolleinheit 31 an die relevanten Elemente. Anschließend erfolgt die physikalische Umsetzung der Einstellparameter in den Elementen und die Partikel werden dem Bestrahlungsplatz zugeführt. Dieser Ablauf hat den Vorteil, dass die Umsetzung erst nach erfolgtem Bestätigungssignal vorgenommen wird und eine eventuelle Fehleinstellung somit frühzeitig verhindert werden kann.
Beispielsweise kann im Fall, dass eine nicht anfordernde Kontrolleinheit ein Bestätigungssignal erhält, automatisch ein entsprechende Deaktivierung vorgenommen werden.

Das zuletzt beschriebene Vorgehen lässt sich in drei Stufen untergliedern. In einer ersten Stufe zur Vorbereitung kommunizieren nur die Kontrolleinheit und die Zuordnungseinheit (Strahlanfragesignal, Bestätigungssignal der Strahlzuordnung sowie "Strahl an"-Signal. In einer zweiten Stufe der Einstellung kommunizieren die zugeordnete Kontrolleinheit und die Beschleunigerkontrolleinheit, d.h., es werden entsprechende Strahlparameter angefordert und die entsprechenden Parameter an die Elemente und die Beschleunigereinheit übermittelt. In einer dritten Stufe der Aktivierung kommuniziert die Zuordnungseinheit direkt mit den Elementen und macht die jeweils benötigten Elemente einstellbar, so dass die von der Beschleunigerkontrolleinheit übermittelten Parameter umgesetzt werden können. Die dritte Stufe macht die Einstellung physikalisch möglich und setzt sie um; sie kann auch schon zeitgleich mit der zweiten Stufe erfolgen.

Während des Bestrahlungsvorgangs arbeitet die Therapieanlage vollkommen autark. Z.B. steuert die Kontrolleinheit 33 Scannermagnete und Strahldiagnoseeinheiten zur Überwachung der Strahlqualität. Die einzige Eingriffsmöglichkeit für das Betriebspersonal ist ein Abbruch des Bestrahlungsvorgangs. Im Fall eines ausgelösten Strahlabbruchs oder eines anderweitig erkannten Fehlers im System entzieht die Zuordnungseinheit 35 über die direkten und fest zugeordneten Signalleitungen zu den einstellbaren Elementen die Erlaubnis, aktiviert zu sein. Bei Eintritt eines derartigen Fehlers mit erfolgt z.B. eine Strahlvernichtung innerhalb der Schikane durch Herunterfahren eines Dipolmagnetfeldes. Zusätzlich werden z.B. die Umlenkmagnete 20,21,23 stromlos geschaltet und der KO-Exciter ausgeschaltet.

Nach Abschluss eines Bestrahlungsvorganges können die einstellbaren Elemente wieder auf ihre Default-Werte eingestellt; so werden z.B. die Umlenk- und/oder Schikanenmagnetfelder auf Null gefahren sowie die KO-Frequenz ausgeschaltet. In Wechselwirkung mit einem die Ausnutzung optimierenden Betriebssystems der Therapieanlage zur Steuerung von anfallenden Bestrahlungsvorgängen kann ein Default-Wert evtl. in Hinblick auf den als nächstes erfolgenden Bestrahlungsvorgangs durch entsprechende Steuerung der Zuordnungseinheit übersprungen werden, so dass der Strahlenpfad schneller für den folgenden Bestrahlungsvorgang zur Verfügung steht.

Die Aufgaben der verschiedenen Komponenten des Kontroll- und Sicherheitssystems für die Strahlanforderung und Strahlpfadfestlegung lassen sich wie folgt zusammenfassen:
- Die Beschleunigerkontrolleinheit 31 kontrolliert die richtigen Werte der Einstellparameter für die einstellbaren Elemente in der Beschleuniger- und Strahlzuführungseinheit.
- Die Zuordnungseinheit 35 gewährt die Einstellbarkeit dieser Parameter mittels eines Aktivierungsprozesses, bei dem gezielt nur diejenigen Elemente aktiviert werden, die für einen Strahlpfad notwendig sind. Die Zuordnungseinheit hat hierfür vorzugsweise eine Tabelle mit den möglichen Strahlpfaden in beispielsweise einem Lock-up-Table gespeichert. Zusätzlich erfolgt eine Prüfung der Verfügbarkeit des Partikelstrahls innerhalb der bevorzugt als sicherheitsgerichtete speicherprogrammierbare Steuerung ausgebildeten Zuordnungseinheit 35. Eine Zuteilung des Partikelstrahls erfolgt nur bei dessen Verfügbarkeit.
- Die Kontrolleinheiten in den Bestrahlungsplätzen liefern die Daten aus dem Bestrahlungsplan und entscheiden letztendlich über die Zuführung des Strahls, d.h., sie lösen die Strahlzuführung an dem entsprechenden Bestrahlungsplatz aus.

Die Verwendung der direkten und fest zugeordneten Verbindungen ist nicht auf die in der Figur skizzierte Ausführungsform beschränkt. So kann beispielsweise nur die Verbindung zwischen einer der Kontrolleinheiten und der Zuordnungseinheit derart ausgeführt sein und die Einstellung von Elementen anders sichergestellt werden.

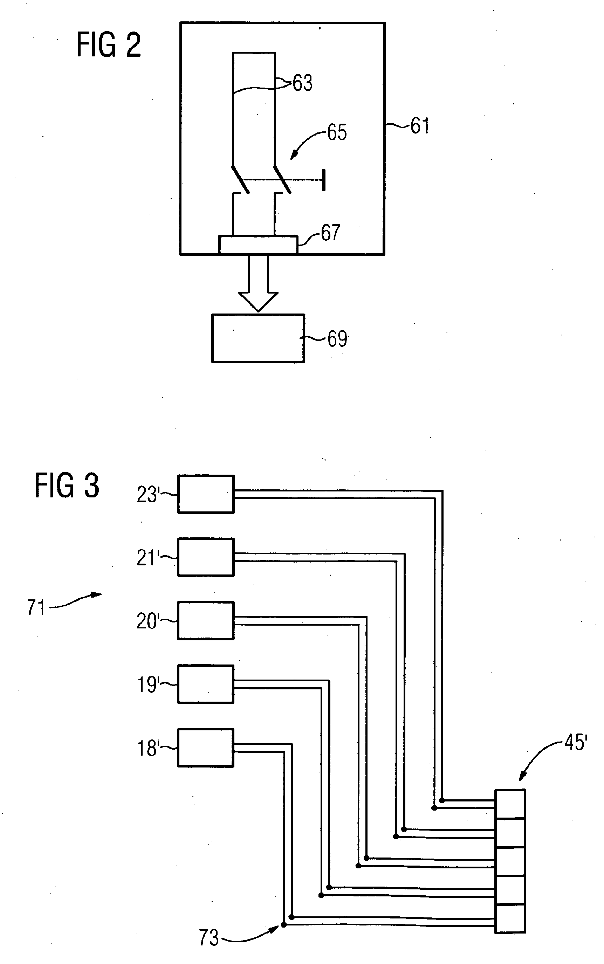

In den Figuren 2 und 3 werden Aspekte einer sicherheitsgerichteten direkten Verbindung verdeutlicht. Figur 2 zeigt schematisch eine sicherheitsgerichtete Schalteinheit 61, wie sie z.B. in der Kontrolleinheit und/oder der Zuordnungseinheit z.B. zur Übermittlung eines Anforderungs-, Bestätigungs- und/oder Aktivierungssignals verwendet werden kann. Zwei parallel geschaltete Leitungen 63 sind über einen zwangsöffnenden/zwangsschließenden Schalter 65 mit einem Signalausgang 67 verbindbar. Der Schalter 65 öffnet/schließt die beiden Leitungen 63 gemeinsam und nimmt im Fehlerfall einen sicheren Zustand ein. Der Signalausgang ist mit einer Einheit 69 verbunden; d.h. übertragen auf Figur 1 ist die Einheit 69 z.B. eine der Kontrolleinheiten 33, die Zuordnungseinheit 35 oder eines der aktivierbaren Elemente wie Auskoppeleinheit 18, Schikane 19 oder einer der Umlenkmagnete 20, 21, 23.

Figur 3 verdeutlicht den Einsatz von sicherheitsgerichteten Doppel-Leitungen für die Übermittlung von Aktivierungssignalen an einzustellenden Elemente 71 wie eine Auskoppeleinheit 18', eine Schikane 19' und Umlenkmagnete 20', 21', 23'. Diese werden von entsprechenden sicherheitsgerichtete Schalteinheit an Signalausgängen 45' angelegt, welche gemäß Figur 1 Teil der Zuordnungseinheit sind. Evtl. ist der Einsatz von Aufklemmungen 73 aufgrund der Größe einer Therapieanlage nicht vermeidbar.

## Patentansprüche

1. Partikeltherapieanlage (1) mit einer Beschleuniger- und Partikelstrahlzuführungseinheit (3, 5) zur Beschleunigung von Partikeln und zur Zuführung von Partikeln an mindestens zwei Bestrahlungsplätzen (11, 13, 15, 17) und mit einem Kontroll- und Sicherheitssystem zur Sicherstellung und Überwachung der korrekten Partikelstrahlführung entlang eines Strahlpfades, wobei das Kontroll- und Sicherheitssystem eine Zuordnungseinheit (35), mindestens eine an einem der Bestrahlungsplätze (11, 13, 15, 17) angeordnete Kontrolleinheit (33) und eine Beschleunigerkontrolleinheit (31) aufweist,
- wobei die Kontrolleinheit (33) zur Abgabe eines Anforderungssignals zur Anforderung eines Partikelstrahls für einen Bestrahlungsvorgang ausgebildet ist,
- wobei die Zuordnungseinheit (35) zur Zuordnung des Partikelstrahls zu dem anfordernden Bestrahlungsplatz ausgebildet ist und mit einstellbaren Elementen (18, 19, 20, 21, 23) der Beschleuniger- und Partikelstrahlzuführungseinheit (3, 5) zur Einstellung des Strahlpfades verbunden ist, um deren Einstellbarkeit mittels eines von der Beschleunigerkontrolleinheit entkoppelten Aktivierungsprozesses zu kontrollieren, wobei gezielt nur diejenigen Elemente mit Hilfe eines direkt von der Zuordnungseinheit zu den Elementen gesendeten Aktivierungssignals aktiviert werden, die für den Strahlpfad notwendig sind, und
- wobei die Beschleunigerkontrolleinheit (31) zur Abgabe von Einstellparametern an die einstellbaren Elemente (18, 19, 20, 21, 23) ausgebildet ist und wobei eine Umsetzung der Einstellparameter nur bei Vorliegen des Aktivierungssignals der Zuordnungseinheit erfolgen kann.

2. Partikeltherapieanlage nach Anspruch 1, wobei die Kontrolleinheit (33) ferner zur Übermittlung von Daten aus einem Bestrahlungsplan ausgebildet ist.

3. Partikeltherapieanlage nach Anspruch 1 oder 2, wobei die Kontrolleinheit (33) ferner zur Auslösung eines den Strahl freigebenden Signals ausgebildet ist, das die Strahlzuführung an dem entsprechenden Bestrahlungsplatz auslöst.

4. Partikeltherapieanlage nach einem der Ansprüche 1 bis 3, wobei die Kontrolleinheit (33) über eine erste fest zugeordnete Signalverbindung (37A ... 37C) direkt mit einem Signaleingang der Zuordnungseinheit (35) verbunden ist, so dass ein Anliegen des Anforderungssignals am Signaleingang eindeutig den anfordernden Bestrahlungsplatz festlegt.

5. Partikeltherapieanlage nach einem der Ansprüche 1 bis 4, wobei die Zuordnungseinheit (35) zur Prüfung einer Verfügbarkeit des Partikelstrahls und bevorzugt als sicherheitsgerichtete speicherprogrammierbare Steuerung ausgebildet ist.

6. Partikeltherapieanlage nach einem der Ansprüche 1 bis 5, wobei die Zuordnungseinheit (35) zur Speicherung einer Tabelle mit den möglichen Strahlpfaden ausgebildet ist.

7. Partikeltherapieanlage nach einem der Ansprüche 1 bis 6, wobei die Beschleunigerkontrolleinheit (31) zur Kontrolle der richtigen Einstellung der einstellbaren Elemente (18, 19, 20, 21, 23) für die angeforderte Strahlzuführung ausgebildet ist.

8. Partikeltherapieanlage nach einem der Ansprüche 1 bis 7, wobei zur Übermittlung eines Bestätigungssignals zwischen der Zuordnungseinheit (35) und der Kontrolleinheit (33) des Bestrahlungsplatzes eine zweite fest zugeordnete Signalverbindung (39A ... 39C) von einem Signalausgang der Zuordnungseinheit (35) zur Kontrolleinheit (33) oder eine gemeinsame Anbindung an ein Datenbus-System (41, 43) besteht.

9. Partikeltherapieanlage nach einem der Ansprüche 1 bis 8, wobei eine der Signalverbindungen eine direkte Hardware-Verbindung, insbesondere eine einzelne Signalleitung, ist.

10. Partikeltherapieanlage nach einem der Ansprüche 1 bis 9, **dadurch gekennzeichnet, dass** mindestens zwei Kontrolleinheiten (33) einzeln über je eine fest zugeordnete Signalverbindung (37A ... 37C) direkt mit je mindestens einem Signalausgang der Zuordnungseinheit (35) verbunden sind.

11. Partikeltherapieanlage nach einem der Ansprüche 1 bis 10, wobei die Beschleunigerkontrolleinheit (31) und die mindestens eine Kontrolleinheit (33) zum Austausch von zur Bestrahlung benötigten Parametern des Partikelstrahls und/oder der Beschleuniger- und Partikelstrahlzuführungseinheit (3, 5), welche insbesondere vom Kontrollsystem aufgrund eines dem Bestrahlungsvorgang zugrunde liegenden Therapieplans (51) festgelegt sind, an einem Datenbus-System (41, 43) angebunden sind.

12. Partikeltherapieanlage nach einem der Ansprüche 1 bis 11, wobei mindestens einer der Bestrahlungsplätze ein Behandlungsplatz (11, 13, 15) ist, an dem ein Patient mit den Partikeln bestrahlt wird.

13. Partikeltherapieanlage nach einem der Ansprüche 1 bis 12, wobei mindestens einer der Bestrahlungsplätze ein Überprüfungsplatz (17) zur Überprüfung von die Partikelbestrahlung charakterisierenden Parametern ist.

14. Verfahren zur Ausbildung eines Strahlpfads für einen Bestrahlungsvorgang in einer Partikeltherapieanlage (1) mit einer Beschleuniger- und Partikelstrahlzuführungseinheit (3, 5) zur Beschleunigung von Partikeln und zur Zuführung der Partikel an mindestens zwei Bestrahlungsplätzen (11, 13, 15, 17) und mit einem Kontroll- und Sicherheitssystem zur Sicherstellung und Überwachung der korrekten Partikelstrahlführung entlang des Strahlpfades, wobei das Kontroll- und Sicherheitssystem eine Zuordnungseinheit (35), je eine jeweils an den Bestrahlungsplätzen (11, 13, 15, 17) angeordnete Kontrolleinheit (33) und eine Beschleunigerkontrolleinheit (31) aufweist, mit folgenden Verfahrensschritten:
- ein Anfrageschritt, in dem eine der Kontrolleinheiten (33) und die Zuordnungseinheit (35) zur Einleitung des Bestrahlungsvorgangs kommunizieren, wobei ein Strahlanfragesignal und nach einer Zuordnung des Partikelstrahls zum anfordernden Bestrahlungsplatz ein die Anfrage bestätigendes Bestätigungssignal ausgetauscht werden,
- ein Einstellungsschritt, in dem die zugeordnete Kontrolleinheit (33) die Beschleunigerkontrolleinheit (31) über den durchzuführenden Bestrahlungsvorgang informiert und in dem die Beschleunigerkontrolleinheit (31) entsprechende Einstellparameter an einstellbare Elemente (18, 19, 20, 21, 23) der Beschleuniger- und Strahlführungseinheit (3, 5) übermittelt,
- einen von der Beschleunigerkontrolleinheit entkoppelten Aktivierungsschritt, in dem die Zuordnungseinheit (35) direkt die jeweils benötigten Elemente mit Hilfe eines direkt von der Zuordnungseinheit zu den Elementen gesendeten Aktivierungssignals aktiviert, so dass die von der Beschleunigerkontrolleinheit (31) übermittelten Einstellparameter umgesetzt werden, so dass der Strahlpfad für die Partikel zum anfordernden Bestrahlungsplatz ausgebildet wird, und wobei eine Umsetzung der Einstellparameter nur bei Vorliegen des Aktivierungssignals der Zuordnungseinheit erfolgen kann.

15. Verfahren nach Anspruch 14, wobei der Aktivierungsschritt zeitgleich mit der Einstellungsstufe erfolgt.

16. Verfahren nach Anspruch 14 oder 15, wobei im Anforderungsschritt ferner ein "Strahl an"-Signal an die Zuordnungseinheit (35) übermittelt wird, dessen Vorliegen in der Zuordnungseinheit (35) zur Durchführung des Aktivierungsschritts vorausgesetzt ist.

## Claims

1. Particle therapy facility (1) including an accelerator and particle beam delivery unit (3, 5) for accelerating particles and delivering particles to at least two irradiation positions (11, 13, 15, 17) and including a control and safety system for ensuring and monitoring the correct particle beam flow along a beam path, wherein the control and safety system has an assignment unit (35), at least one control unit (33) which is arranged at one of the irradiation positions (11, 13, 15, 17) and an accelerator control unit (31),
- wherein the control unit (33) is designed for emitting a request signal in order to request a particle beam for an irradiation procedure,
- wherein the assignment unit (35) is designed for assigning the particle beam to the requesting irradiation position and is connected to configurable elements (18, 19, 20, 21, 23) of the accelerator and particle beam delivery unit (3, 5) for configuring the beam path, in order to control their configurability by means of an activation process that is decoupled from the accelerator control unit, wherein only those elements which are required for the beam path are selectively activated with the aid of an activation signal sent directly from the assignment unit to the elements, and
- wherein the accelerator control unit (31) is designed for emitting configuration parameters to the configurable elements (18, 19, 20, 21, 23) and wherein the configuration parameters can only be implemented in the presence of the activation signal from the assignment unit.

2. Particle therapy facility according to claim 1, wherein the control unit (33) is also designed for transferring data from an irradiation schedule.

3. Particle therapy facility according to claim 1 or 2, wherein the control unit (33) is also designed for triggering a signal which enables the beam, which signal triggers the beam delivery at the corresponding irradiation position.

4. Particle therapy facility according to one of claims 1 to 3, wherein the control unit (33) is directly connected to a signal input of the assignment unit (35) via a first permanently assigned signal connection (37A ... 37C), such that the presence of the request signal at the signal input unambiguously specifies the requesting irradiation position.

5. Particle therapy facility according to one of claims 1 to 4, wherein the assignment unit (35) is designed for checking an availability of the particle beam, and preferably as a fail-safe stored-program control system.

6. Particle therapy facility according to one of claims 1 to 5, wherein the assignment unit (35) is designed for storing a table containing the possible beam paths.

7. Particle therapy facility according to one of claims 1 to 6, wherein the accelerator control unit (31) is designed for controlling the correct configuration of the configurable elements (18, 19, 20, 21, 23) for the requested beam delivery.

8. Particle therapy facility according to one of claims 1 to 7, wherein a second permanently assigned signal connection (39A ... 39C) from a signal output of the assignment unit (35) to the control unit (33) or a shared access to a data bus system (41, 43) exists for transferring a confirmation signal between the assignment unit (35) and the control unit (33) of the irradiation position.

9. Particle therapy facility according to one of claims 1 to 8, wherein one of the signal connections is a direct hardware connection, in particular an individual signal line.

10. Particle therapy facility according to one of claims 1 to 9,
**characterised in that** at least two control units (33) are individually connected, each via a permanently assigned signal connection (37A ... 37C) and each directly to at least one signal output of the assignment unit (35).

11. Particle therapy facility according to one of claims 1 to 10, wherein the accelerator control unit (31) and the at least one control unit (33) are connected to a data bus system (41, 43) for the exchange of parameters of the particle beam which are required for the irradiation and/or parameters of the accelerator and particle beam delivery unit (3, 5), which parameters are specified in particular by the control system with reference to a therapy schedule (51) which forms the basis of the irradiation procedure.

12. Particle therapy facility according to one of claims 1 to 11, wherein at least one of the irradiation positions is a treatment position (11, 13, 15) at which a patient is irradiated with the particles.

13. Particle therapy facility according to one of claims 1 to 12, wherein at least one of the irradiation positions is a test position (17) for checking parameters which characterise the particle irradiation.

14. Method for forming a beam path for an irradiation procedure in a particle therapy facility (1) which includes an accelerator and particle beam delivery unit (3, 5) for accelerating particles and delivering the particles to at least two irradiation positions (11, 13, 15, 17) and includes a control and safety system for ensuring and monitoring the correct particle beam flow along the beam path, wherein the control and safety system features an assignment unit (35), one control unit (33) arranged at each of the irradiation positions (11, 13, 15, 17) and an accelerator control unit (31), comprising the following method steps:
- a request step, in which one of the control units (33) and the assignment unit (35) communicate in order to initiate the irradiation procedure, wherein a beam request signal and, following an assignment of the particle beam to the requesting irradiation position, a confirmation signal confirming the request are exchanged,
- a configuration step, in which the assigned control unit (33) informs the accelerator control unit (31) about the irradiation procedure which must be carried out, and in which the accelerator control unit (31) transfers corresponding configuration parameters to configurable elements (18, 19, 20, 21, 23) of the accelerator and beam delivery unit (3, 5),
- an activation step that is decoupled from the accelerator control unit, in which the assignment unit (35) directly activates the required elements in each case with the aid of an activation signal sent directly from the assignment unit to the elements, such that the configuration parameters which were transferred from the accelerator control unit (31) are implemented and therefore the beam path is formed for the particles to the requesting irradiation position and wherein the configuration parameters can only be implemented in the presence of the activation signal from the assignment unit.

15. Method according to claim 14, wherein the activation step takes place concurrently with the configuration stage.

16. Method according to claim 14 or 15, wherein a "Beam on" signal is additionally transferred to the assignment unit (35) in the request step, the presence of said signal in the assignment unit (35) being a prerequisite for the execution of the activation step.

## Revendications

1. Système de thérapie par particules (1) comprenant un ensemble d'accélérateur et de guidage de faisceau de particules (3, 5) destiné à accélérer des particules et à guider des particules sur au moins deux postes d'irradiation (11, 13, 15, 17), et comprenant un système de contrôle et de sécurité destiné à assurer et surveiller le guidage correct du faisceau de particules le long d'une trajectoire de faisceau, le système de contrôle et de sécurité comprenant une unité d'affectation (35), au moins une unité de contrôle (33) disposée sur l'un des postes d'irradiation (11, 13, 15, 17) et une unité de contrôle de l'accélérateur (31),
- dans lequel l'unité de contrôle (33) est conçue pour émettre un signal de demande destiné à demander un faisceau de particules pour un processus d'irradiation,
- dans lequel l'unité d'affectation (35) est conçue pour affecter le faisceau de particules au poste d'irradiation demandeur et est reliée à des éléments réglables (18, 19, 20, 21, 23) de l'ensemble d'accélérateur et de guidage de faisceau de particules (3, 5), destinés à régler la trajectoire de faisceau, afin de contrôler leur réglage au moyen d'un processus d'activation découplé de l'unité de contrôle de l'accélérateur, seuls les éléments nécessaires pour la trajectoire du faisceau étant activés de manière ciblée à l'aide d'un signal d'activation, envoyé directement de l'unité d'affectation aux éléments, et
- dans lequel l'unité de contrôle de l'accélérateur (31) est conçue pour délivrer des paramètres de réglage aux éléments réglables (18, 19, 20, 21, 23), une mise en oeuvre des paramètres de réglage ne pouvant avoir lieu qu'en présence du signal d'activation de l'unité d'affectation.

2. Système de thérapie par particules selon la revendication 1, dans lequel l'unité de contrôle (33) est conçue en outre pour transmettre des données issues d'un plan d'irradiation.

3. Système de thérapie par particules selon la revendication 1 ou 2, dans lequel l'unité de contrôle (33) est conçue en outre pour déclencher un signal qui libère le faisceau et qui déclenche le guidage du faisceau sur le poste d'irradiation correspondant.

4. Système de thérapie par particules selon l'une des revendications 1 à 3, dans lequel l'unité de contrôle (33) est reliée via une première liaison de signal dédiée (37A ... 37C) directement à une entrée de signal de l'unité d'affectation (35) de sorte qu'une application du signal de demande à l'entrée de signal détermine clairement le poste d'irradiation demandeur.

5. Système de thérapie par particules selon l'une des revendications 1 à 4, dans lequel l'unité d'affectation (35) est conçue pour vérifier une disponibilité du faisceau de particules et se présente de préférence sous forme de commande à mémoire programmable orientée sécurité.

6. Système de thérapie par particules selon l'une des revendications 1 à 5, dans lequel l'unité d'affectation (35) est conçue pour mémoriser une table contenant les possibles trajectoires du faisceau.

7. Système de thérapie par particules selon l'une des revendications 1 à 6, dans lequel l'unité de contrôle de l'accélérateur (31) est conçue pour contrôler le bon réglage des éléments réglables (18, 19, 20, 21, 23) pour le guidage demandée du faisceau.

8. Système de thérapie par particules selon l'une des revendications 1 à 7, dans lequel il existe pour la transmission d'un signal de confirmation entre l'unité d'affectation (35) et l'unité de contrôle (33) du poste d'irradiation, une seconde liaison de signal dédiée (39A ... 39C) allant d'une sortie de signal de l'unité d'affectation (35) à l'unité de contrôle (33), ou une connexion commune à un système de bus de données (41, 43).

9. Système de thérapie par particules selon l'une des revendications 1 à 8, dans lequel l'une des liaisons de signal est une liaison matérielle directe, en particulier une liaison de signal individuelle.

10. Système de thérapie par particules selon l'une des revendications 1 à 9, **caractérisé en ce qu'**au moins deux unités de contrôle (33) sont reliées individuellement, chacune via une liaison de signal dédiée (37A ... 37C), directement à au moins une sortie de signal de l'unité d'affectation (35).

11. Système de thérapie par particules selon l'une des revendications 1 à 10, dans lequel l'unité de contrôle de l'accélérateur (31) et ladite au moins une unité de contrôle (33) sont connectées à un système de bus de données (41, 43) pour échanger des paramètres du faisceau de particules et/ou de l'ensemble d'accélérateur et de guidage de faisceau de particules (3, 5), qui sont nécessaires pour l'irradiation et qui sont notamment déterminés par le système de contrôle en considération d'un plan de thérapie (51) qui est à la base du processus d'irradiation.

12. Système de thérapie par particules selon l'une des revendications 1 à 11, dans lequel au moins l'un des postes d'irradiation est un poste de traitement (11, 13, 15) où un patient est irradié avec les particules.

13. Système de thérapie par particules selon l'une des revendications 1 à 12, dans lequel au moins l'un des postes d'irradiation est un poste de contrôle (17) destiné à contrôler des paramètres caractérisant l'irradiation par particules.

14. Procédé de formation d'une trajectoire de faisceau pour un processus d'irradiation dans un système de thérapie par particules (1) comprenant un ensemble d'accélérateur et de guidage de faisceau de particules (3, 5) destiné à accélérer des particules et à guider les particules sur au moins deux postes d'irradiation (11, 13, 15, 17), et comprenant un système de contrôle et de sécurité destiné à assurer et surveiller le guidage correct du faisceau de particules le long de la trajectoire de faisceau, le système de contrôle et de sécurité comprenant une unité d'affectation (35), une unité de contrôle (33) disposée sur chacun des postes d'irradiation (11, 13, 15, 17) et une unité de contrôle de l'accélérateur (31), comprenant les étapes suivantes :
- une étape de demande pendant laquelle l'une des unités de contrôle (33) et l'unité d'affectation (35) communiquent pour déclencher le processus d'irradiation, et pendant laquelle un signal de demande de faisceau et, après une affectation du faisceau de particules au poste d'irradiation demandeur, un signal de confirmation de la demande sont échangés,
- une étape de réglage pendant laquelle l'unité de contrôle affectée (33) informe l'unité de contrôle de l'accélérateur (31) sur le processus d'irradiation à effectuer, et pendant laquelle l'unité de contrôle de l'accélérateur (31) transmet des paramètres de réglage correspondants à des éléments réglables (18, 19, 20, 21, 23) de l'ensemble d'accélérateur et de guidage de faisceau (3, 5),
- une étape d'activation, découplée de l'unité de contrôle de l'accélérateur, pendant laquelle l'unité d'affectation (35) active directement les éléments correspondants nécessaires à l'aide d'un signal d'activation envoyé directement de l'unité d'affectation aux éléments, de sorte que les paramètres de réglage transmis par l'unité de contrôle de l'accélérateur (31) soient mis en oeuvre de manière à former la trajectoire de faisceau pour les particules vers le poste d'irradiation demandeur, et dans laquelle une mise en oeuvre des paramètres de réglage ne peut avoir lieu qu'en présence du signal d'activation de l'unité d'affectation.

15. Procédé selon la revendication 14, dans lequel l'étape d'activation a lieu en même temps que l'étape de réglage.

16. Procédé selon la revendication 14 ou 15, dans lequel, pendant l'étape de demande, un signal « faisceau allumé » est transmis en outre à l'unité d'affectation (35), signal dont la présence dans l'unité d'affectation (35) est supposée pour effectuer l'étape d'activation.
